(19)

Europäisches
Patentamt
European
Patent Office
Office européen
des brevets

(11) **EP 2 161 029 A1**

(12) **EUROPEAN PATENT APPLICATION**

(43) Date of publication:
**10.03.2010 Bulletin 2010/10**

(51) Int Cl.:
*A61K 38/05* (2006.01)    *A61P 9/12* (2006.01)

(21) Application number: **08163908.0**

(22) Date of filing: **09.09.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(71) Applicants:
• **Unilever N.V.
3013 AL Rotterdam (NL)**
Designated Contracting States:
**AT BE BG CH CZ DE DK EE ES FI FR GR HR HU
IS IT LI LT LU LV MC NL NO PL PT RO SE SI SK TR**
• **Unilever PLC
London
EC4Y 0DY (GB)**
Designated Contracting States:
**CY GB IE MT**

(72) Inventors:
• **van Buren, Leendert
3133 AT Vlaardingen
(NL)**
• **Foltz, Martin
3133 AT Vlaardingen (NL)**
• **Ramcharan, Jane Rita
3133 AT Vlaardingen (NL)**

(74) Representative: **Corsten, Michael Allan et al
Unilever Patent Group
Olivier van Noortlaan 120
3133 AT Vlaardingen (NL)**

(54)    **Composition comprising peptides**

(57)    Food product suitable for the prevention or treatment of hypertension comprising one or more peptides wherein the N-terminal amino acid is D, G or P and wherein said peptides are selected from the group consisting of the dipeptides DW, PW, GC and GN.

EP 2 161 029 A1

**Description**

**Field of the invention**

The present invention concerns a food product comprising peptides which have a blood pressure lowering effect when the food product is used according to the common needs of the consumer.

**Background prior art**

Hypertension or high blood pressure is considered to be one of the main risk factors for Cardio Vascular Diseases (CVD). One of the mechanisms which regulates blood pressure is the renin-angiotensin system. This is a cascade of reactions leading to the formation of angiotensin II, which has a strong vasoconstrictive and hence blood pressure increasing effect. Inhibition of one of the key enzymes in this cascade: Angiotensin I Converting Enzyme (ACE) reduces formation of angiotensin II and thus has a blood pressure lowering effect. Long term human intervention studies have shown regular intake of low amounts of ACE inhibitors reduces CVD by 25% (Gerstein et al. (2000), The Lancet 355, 253-259).

ACE-inhibitors in food products are well known. Such food products have for instance been prepared by fermentation of milk or milk products. In a placebo-controlled study, the blood pressure lowering effect of sour milk containing the ACE-inhibiting peptides VPP and IPP was shown in hypertensive humans (Hata, Y et al. (1996), American Journal of Clinical Nutrition 64, 767-771).

A commercially available fermented milk product, which claims to be "suitable for those with mild hypertension" is Calpis sour milk, fermented with Lactobacillus helveticus and Saccharomyces cervisiae, produced by Calpis Food Industry, Japan. Another commercially available fermented milk product is Evolus produced by Valio, Finland. These fermented milk products are fermented with Lactobacillus helveticus (Lb. helveticus) strains. The products contain tripeptides (VPP and IPP) responsible for in vitro ACE inhibition.

The known products have a blood pressure lowering effect. It is known that a long term blood pressure lowering results in a reduction of risk of CVD.

Protein hydrolysates or fermentates may contain particular peptides that decrease blood pressure, probably by their inhibitory activity on ACE. The observed blood pressure lowering effects are usually modest. Therefore, there remains a need to formulate alternative food products which can help to treat or prevent hypertension. In particular there remains a need to identify those active components for incorporation in food, especially di- or tripeptides which have (a) an adequate Ace inhibiting activity, and (b) are preferably stable under the storage conditions of the food, and (c) are preferably stable under conditions in the gastrointestinal tract, and (d) are capable of passing through membranes to enter into the bloodstream.

Surprisingly it has been found that the specific selection of peptides, preferably in defined amounts, preferably in combination with defined food products leads to a combination which satisfies, at least partly, the criteria (a) - (d) as indicated above.

**Summary of the invention**

Accordingly, in a first aspect, the invention relates to a food product suitable for the prevention or treatment of hypertension comprising one or more peptides wherein the N-terminal amino acid is D, G or P and wherein said peptides are selected from the group consisting of the dipeptides DW, PW, GC and GN.

In a second aspect the invention relates to said food product for use in the prevention or treatment of hypertension.

**Detailed description of the invention**

Peptides are short polymers formed from the linking, in a defined order, of $\alpha$-amino acids, for example two (called a dipeptide) or three (called tripeptide) amino acids. The link between one amino acid residue and the next is known as an amide bond or a peptide bond.

An amino acid is a molecule containing both amine and carboxyl functional groups. In biochemistry, this term refers to alpha-amino acids with the general formula H2NCHRCOOH, where R is an organic substituent. In the alpha amino acids, the amino and carboxylate groups are attached to the same carbon, which is called the $\alpha$-carbon. The various alpha amino acids differ in which side chain (R group) is attached to their alpha carbon. Examples of amino acids are Alanine (A), Valine (V), Leucine (L), Isoleucine (I), Proline (P), Tryptophan (W), Phenylalanine (F), Methionine (M), Glycine (G), Serine (S), Threonine (T), Tyrosine (Y), Cysteine (C), Glutamine (Q), Asparagine (N), Lysine (K), Arginine (R), Histidine (H), Aspartic acid (D) and Glutamic acid (E).

The amino acids for a given peptide are usually notated from left to right wherein the leftmost amino acid (i.e. the first amino acid) is called the N-terminal amino acid and the rightmost amino acid on the right (i.e. the last amino acid) is called the C-terminal amino acid.

The carboxyl group of the N-terminal amino acid forms a peptide bond with the amine group of the next amino acid. Likewise, the amine group of the C-terminal amino acid forms a peptide bond with the carboxyl group of the amino acid preceding it.

For example, PA denotes a dipeptide wherein P is the N-terminal amino acid and A is the C-terminal amino acid. The carboxyl group of P forms a peptide bond with the amine group of A.

ALS, denotes tripeptide wherein A is the N-terminal amino acid and S is the C-terminal amino acid. The carboxyl group of A forms a peptide bond with the amine group of L and the amine group of S forms a peptide bond with the carboxyl group of L.

Peptides for use in the food products according to the invention are not beta-peptides. Preferably the peptides contain amino acids commonly present in naturally occurring peptides. Preferably the peptides are alpha-peptides.

Surprisingly, it was found that peptides wherein the N-terminal amino acid is D, G or P are more stable, that is such peptides are less prone to degradation during gastrointestinal passage and absorption, more specifically that such peptides exhibit stability under simulated small intestinal conditions.

Preferably the peptides for use in the food products according to the invention are dipeptides selected from the group consisting of DW, PW, GC and GN.

The peptides may be obtained or manufactured by any method known in the art, including synthesis of the pure peptides and (isolation from) protein fractions generated by hydrolysis or fermentation. The peptides of the invention may be present in the food product as the peptides per se (e.g. from synthesizing the pure peptides) or as part of a protein fraction (e.g. hydrolyzed or fermented protein).

Food products according to the invention preferably are selected from the group consisting of drinks, dairy type products, frozen confectionery products and spreads/margarine. The amount of the peptides according to the invention in the food product is preferably at least 1 mg/kg, more preferably from 10 to 200, even more preferably from 20 to 100 and most preferably from 30 to 80 mg/kg of said peptides, for example 60 mg/kg.

These preferred types of food products are described in some detail below.

Preferably the amount of peptides in these food products is chosen such that the amount of peptide per total amount of serving of the food product is from 1 to 200 mg, preferably 2 to 100, more preferably 3 to 40 and most preferably 5 to 20 mg.

Suitable serving sizes are fruit juice - 200 gram; milk type drink - 125 gram; yoghurt - 125 gram; frozen confectionary product - 75 gram; spread/margarine - 15 gram; sauce - 40 gram; bar - 60 gram.

Fruit juice products

Examples of fruit juice products according to the invention are juices derived from citrus fruit like orange and grapefruit, tropical fruits, banana, peach, peer, strawberry, to which the peptides and optionally one or more heart health ingredients are added.

Dairy type products

Examples of dairy products according to the invention are milk, dairy spreads, cream cheese, milk type drinks and yogurt, to which the peptides and optionally one or more heart health ingredients are added.

The food product may be used as such as a milk type drink. Alternatively flavor or other additives may be added. A dairy type product may also be made by adding the peptides to water or to a dairy product.

An example of a composition for a yoghurt type product is about 50-80 wt% water, 1-10 wt% of the peptides and optionally one or more heart health ingredients, 0-15 wt% whey powder, 0-15 wt% sugar (e.g. sucrose), 0.01-1 wt% yoghurt culture, 0-20 wt% fruit, 0.05-5 wt% vitamins and minerals, 0-2 wt% flavor, 0-5 wt% stabilizer (thickener or gelling agent). To the yoghurt, fruit may be added.

A typical serving size for a yoghurt type product could be from 50 to 250 g, generally from 80 to 200 g.

Frozen Confectionery Products

For the purpose of the invention the term frozen confectionery product includes milk containing frozen confections such as ice-cream, frozen yoghurt, sherbet, sorbet, ice milk and frozen custard, water-ices, granitas and frozen fruit purees.

Preferably the level of solids in the frozen confection (e.g. sugar, fat, flavoring etc) is more than 3 wt%, more preferred from 10 to 70 wt%, for example 40 to 70 wt%.

Ice cream will typically comprise 0 to 20 wt% of fat, 1 to 10 wt% of the peptides and optionally one or more heart health ingredients, sweeteners, 0 to 10 wt% of non-fat milk components and optional components such as emulsifiers, stabilizers, preservatives, flavoring ingredients, vitamins, minerals, etc, the balance being water. Typically ice cream will be aerated e.g. to an overrun of 20 to 400 %, more specific 40 to 200 % and frozen to a temperature of from -2 to - 200 degrees Celsius, more specific -10 to -30 degrees Celsius. Ice cream normally comprises calcium at a level of about 0.1 wt%.

Oil and water containing emulsions

Advantageously the food product is an oil and water containing emulsion, for instance a spread or a margarine. Oil and water emulsion is herein defined as an emulsion comprising oil and water and includes oil in water (O/W) emulsions and water in oil emulsions (W/O) and more complex emulsions for instance water-in-oil-in-water (W/O/W/O/W) emulsions. Oil is herein defined as including fat.

Preferably a spread according to the invention comprises 30-90 wt% vegetable oil, 1-5 wt% of the peptides, and optionally one or more further heart health ingredients in suitable amounts. Advantageously a spread has a pH of 4.2-6.0.

Most preferably the emulsion is a spreadable water in oil emulsion.

An example of a composition of a spread is 37.45 wt% of a fat blend, 52.8 wt% water, 0.15 wt% lecithin, 0.2 wt% monoglyceride, 0.1 wt% flavour, 0.5 wt% sodium chloride, 0.1 wt% potassium sorbate, 0.1 wt% sweet buttermilk powder, 6 wt% starch, 2.5 wt% of the peptides.

Other food products

Other food products according to the invention can be prepared by the skilled person based on common general knowledge, the peptides and optionally one or more heart health ingredients in suitable amounts. Examples of such food products are baked goods, snacks, sauces, bars etc.

In a second aspect the invention concerns a food product as described herein for use in the prevention or treatment of hypertension.

The invention will be further illustrated by the following non-limiting examples.

**Examples**

Peptides

A peptide library consisting of 228 dipeptides was purchased from JPT Peptide Technologies GmbH (Berlin, Germany). The peptides were synthesized using solid phase Fmoc strategy. Dipeptide purity was >85% and all peptides contained <5% tripeptide contamination as determined with HPLC-UV (Waters, Atlantis Hilic Silica column, Milford MA, USA). Peptide identification was performed by means of LC-MRM-MS. IPP (purity 92.1 %) was obtained from Bachem (Bubendorf, Switzerland). Hippuryl-Histidine-Leucine (HHL) and hippuric acid (HA) was purchased from Sigma-Aldrich Chemie BV (Zwijndrecht, The Netherlands).

Chemicals

NaHC03, pancreatin from porcine pancreas, bile extract and PEG 300, 600, 1000 were obtained from Sigma (St. Louis, USA). NaCl, acetonitrile and methanol, trifluoroacetic acid, formic acid and NH40H were purchased from Merck (Darmstadt, Germany) and hydrochloric acid from Riedel de Haen (Seelze, Germany). ACE was purchased from Sigma-Aldrich Chemie BV (Zwijndrecht, The Netherlands).

Generation of biological data

Peptide databases were constructed for small intestinal peptide stability (228 dipeptides) and ACE inhibition (228 dipeptides) using bioassays. Datasets derived from these bioassays are presented in Supplemental Table 1 and 2.

Simulated small intestinal digestion

The pH of simulated gastric fluid (SGF, 0.15 M NaCl, pH 2.5) was increased to 7.5 with NH4CO3 (40 mM), and the following compounds were added to adjust the pH to 6.8 and simulate a duodenal-jejunal environment and create simulated small intestinal fluid (SIF): (i) a bile salt mixture containing equimolar quantities (0.125 M) of sodium taurocholate and glycodeoxycholic acid in NaHC03 (1 M), (ii) solutions of pancreatin from porcine pancreas (9 mg/mL) in water was prepared and added at approximately physiological ratios. The pH was adjusted to 6.8 with HCl (0.1 M). Peptides were dissolved in SIF at a final peptide concentration of 50 $\mu$M. Aliquots (1 mL) were prepared for sampling at different time points. Simulated intestinal digestion was carried out at 37°C, and aliquots (1 mL) were taken at 0, 5, 15, and 60 min after the start of the experiment. After peptide hydrolysis, digestive enzymes were inactivated at 95°C for 15 min and samples were cooled immediately on ice water and stored at -20 °C until further analysis.

Peptide Quantification

Quantificion of dipeptides derived from digestion were performed using a liquid chromatography multiple reaction ion monitoring-mass spectrometry (LC-MRM-MS) method as described van Platerink CJ, Janssen HG, Horsten R, Haverkamp J. Quantification of ACE inhibiting peptides in human plasma using high performance liquid chromatography-mass spectrometry. J Chromatogr B Analyt Technol Biomed Life Sci 2006,830:151-157.
Peptide separation was performed on an Atlantis Hilic Silica column (2.1 x 150 mm, 3 $\mu$m particle size) and a Micromass Quattro-II (Milford MA, USA) with HP1100LC system (Hewlett-Packard, USA). The gradient started at 100% 0.1% trifluoro acetic acid in Milli-Q water and ended at 70% 0.1 % trifluoro acetic acid in acetonitrile. To support the ionization, a 70/30 mixture of propionic acid and propanol-2 was added postcolumn at a flow rate of 50 mL·min-1. All analyses were conducted on an Alliance 2795 HPLC (Waters, Milford MA, USA) combined with a Quattro Premier triple quadrupole mass spectrometer from the same supplier.

ACE inhibition measurements

The ACE inhibition experiments were performed with a HPLC-UV at-line assay using the artificial substrate HHL as described in van Platerink CJ, Janssen HG, Haverkamp J. Development of an at-line method for the identification of angiotensin-I inhibiting peptides in protein hydrolysates. J Chromatogr B Analyt Technol Biomed Life Sci 2007; 846 (1-2): 147-154.
Briefly, dipeptide samples (40 $\mu$L) at varying concentrations (20 and 200 $\mu$M) were pipetted into a 96 well plate and 27 $\mu$l of the ACE solution (33.4 mU·mLP-1 P in PBS, pH 7.4) was added to each well and incubated for 5 min on a 96 well plate mixer at 700 RPM. 13 $\mu$L of HHL (0.35 mM, in PBS). After 60 min incubation at 50°C the reaction was stopped on melting ice. 30 $\mu$L of the reaction mixture of each well was injected onto a Chromolith Flash RP18e 25 x 4.6 mm HPLC column (Merck, Darmstadt, Germany) equipped with a 10 x 4.6 mm RP18e guard column (Merck, Darmstadt, Germany). HA and HHLwere monitored at 280 nm and quantified by integration using the TotalChrom software vs. 6.2.1 (Perkin Elmer, Gouda, The Netherlands). ACE inhibition of each peptide at a given concentration was calculated based on the following equitation.

$$ACEI\alpha = (DHw - DH\alpha) / (DHw \times 100)$$

wherein ACEI$\alpha$ is the percentage inhibition of the dipeptide, DHw is the degree of hydrolysis (DH) of HHL to HA in water, DH$\alpha$ is the degree of hydrolysis of HHL to HA for the peptide. DH was calculated by expressing the peak area of HA as a percentage of the sum of the peak areas of HA and HHL.

Quantitative structure activity relationship determination

All in silico screening was performed in Scitegic's Pipeline Pilot version 6.0 (Scitegic Inc., San Diego, USA). Structure Activity Relationship (SAR) models were generated using modules present in Pipeline Pilot. All data were visualised and analysed using Spotfire DecisionSite 8.2 (Spotfire Europe, Göteborg, Sweden).

*Preparation of data sets*

An intestinal stability data set consisting of 228 dipeptides was generated based on the results from the simulated digestion experiments. The area under the time - peptide concentration curve was calculated and used for further analysis and modelling. An ACE inhibitory dipeptide database consisting of 228 dipeptides was constructed. For further analysis the ACE inhibitory activity at a peptide concentration of 200 $\mu$M was used.

*Analysis of the data sets*

The dipeptides were characterized based on overall structural features of the dipeptides and each individual amino acid. The characterization of each individual amino acid by the three Z-scores was calculated by principal component analysis (PCA) from a matrix consisting of 29 physicochemical variables as described in Hellberg S, Sjostrom M, Skagerberg B, Wold S. Peptide Quantitative Structure-Activity-Relationships, A Multivariate Approach. J Med Chem 1987,30:1126-1135. Partial least square regression (PLS) analysis between amino acid descriptors (predictors, X) and experimental parameter (dependent Y) was carried out using Scitegic's Pipeline Pilot version 6.0 (Scitegic Inc., San Diego, USA) using Structure Activity Relationship (SAR) models. All variables were centered and scaled to unit variance prior to the analyses except with specification and thereby all variables had and equal participation in the model. Seventeen different models were created based on the most commonly used peptide descriptors. All models were cross validated by removing 10% of the peptides from each individual dataset based on their structural diversity. Models based on the remaining 90% were tested on their ability to predict the values of the data that was removed. This process was repeated 10 times for each model.

Validation of predicted stability of dipeptides

ACE inhibitory peptides with sequences found within food proteins were synthesized and used as external validation set. Small intestinal stability of 12 dipeptides with predicted low (1-4), neutral (5-8) and high (9-12) intestinal stability was tested according to the above described procedures.

Peptide stability under simulated intestinal conditions

For each peptide the relative stability (in % of initial dipeptide concentration) was plotted against time and the area under the curve (AUC) was calculated. The AUC values ranged from 1 unit (<1% stable over 1 min) to 6000 units (100% stable over 60 minutes). 103 out of 220 dipeptides exhibited an AUC >4500 units. These peptides showed a relative stability of more than 75% remaining dipeptide after 60 min of simulated intestinal digestion. 17 dipeptides, including the dipeptides GI (Gly-Ile), GV (Gly-Val), GA (Gly-Ala), and DP (Asp-Pro), were identified as extremely stable under simulated small intestinal digestion conditions with an AUC of >5900 units.

Modelling of peptide stability using QSAR and amino acid clustering

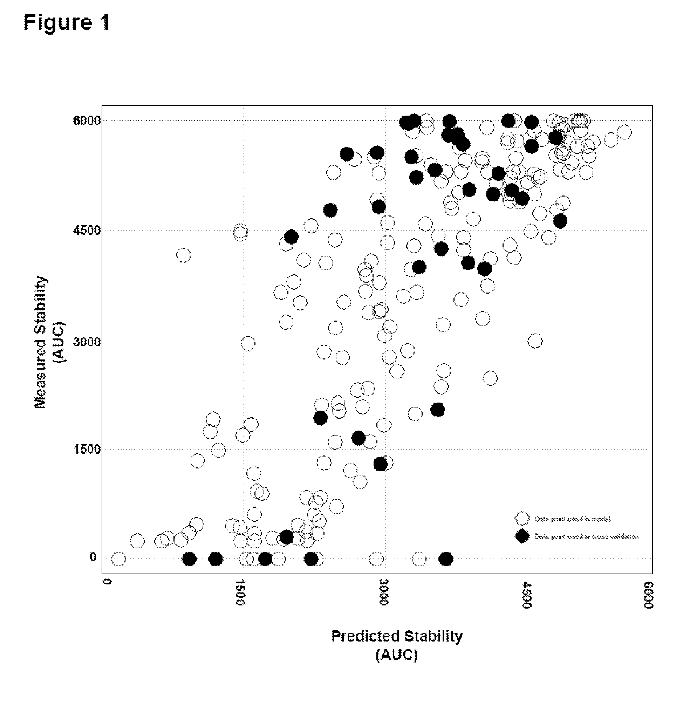

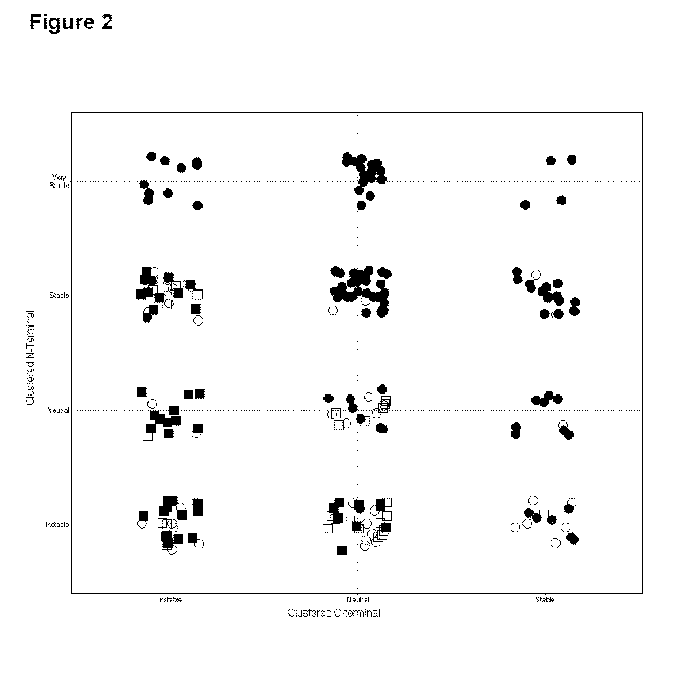

Seventeen different models were created. The generated models were used to predict peptide stability for all measured dipeptides. Predicted stability was plotted against the experimentally measured stability and correlation coefficients were calculated. Correlation coefficients of the predicted stability against the measured stability plots for each model are listed in Table 1. Several models showed high R2 values of >0.65; a model based on the product of the Z and X-scales (model 8) showed the best fit (R2 = 0.76) (Figure 1). The second approach tested to create a predictive model for dipeptide stability was performed by data clustering. The measured dipeptides were divided into groups based on their experimentally measured stability. The groups were clustered based on the contribution of the N-terminal and C-terminal amino acid on the calculated AUC. The contribution of the amino acid residue to the observed dipeptide stability was calculated based on the average AUC of all peptides containing the amino acid of interest. A nearly equal distribution of all N- and C- terminal amino acids was observed in the data set. Peptides were ranked and the N-terminal and C-terminal amino acid residues were then grouped according to the average AUC (Table 2). An N-terminal Gln was considered as small intestinal stable peptide given that Gln is prone to cyclization at low pH to pyro-Glu, which makes the degradation by endopeptidases less effective. Clustering the stability data based on the contribution of the individual amino acid residues into 12 clusters (4 N-terminal clusters and 3 C-terminal clusters) highly correlated with the experimentally measured stability (Figure 2). Based on this, dipeptides were classified into stable, neutral and instable clusters representing the overall stability of the dipeptide (Table 3).

This analysis revealed that dipeptides containing very stable N-terminal amino acid residues, such as D (Asp), G (Gly) and P (Pro), exhibited a high stability under simulated intestinal digestion conditions independent of the C-terminal amino acid residue.

In vitro ACE inhibitory activity of dipeptides

To establish the relationship between dipeptide structure and ACE inhibitory activity 228 dipeptides were assayed for ACE inhibitory activities using HHL as substrate in an HPLC-UV enzyme-based at line assay. A selection of 20 peptides was assayed for their IC50 value using 6 different peptide concentrations. These values ranged from 19 $\pm$ 2 $\mu$M for Ile-Trp to 717 $\pm$ 67 $\mu$M for Gly-Pro (Table 4). IC50 values and % ACE inhibition at a dipeptide concentrations

of 200 μM was highly correlated (R2 = 0.92). ACE inhibitory activity of 228 dipeptides was measured at inhibitor concentrations of 20 and 200 μM. At concentrations of 20 μM, 8 dipeptides reduced ACE activity by >50%, whereas at concentrations of 200 μM 46 dipeptides decreased ACE activity >50 % (Supplemental Table 2).

Structure activity analysis of dipeptides for ACE inhibition

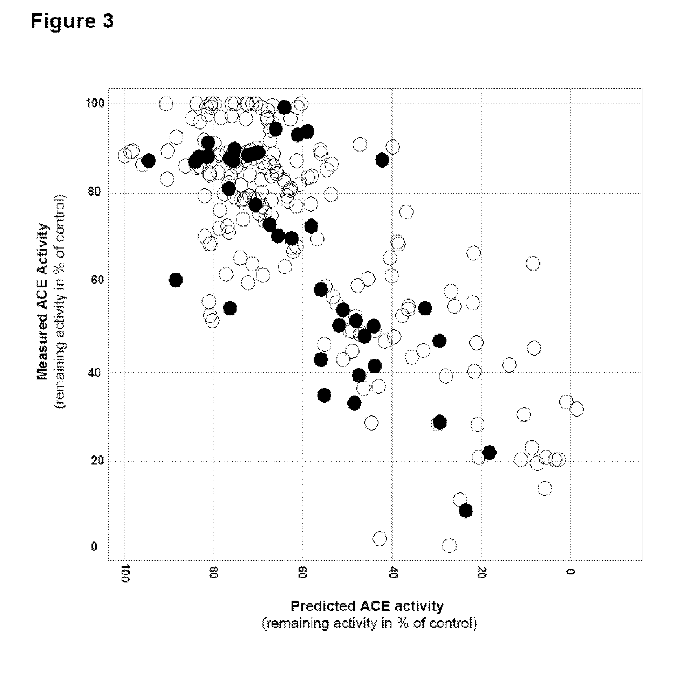

Seventeen different models were constructed as described before. ACE inhibition significantly correlated with a selection of the models indicated by R2 values up to 0.77 (Table 1). In contrast to the stability models, which gave the best correlation for models based on Z and X-scales, ACE inhibitory activity was best described by Scitegic's EC fingerprint models (model 13) (Figure 3).

Prediction and Validation of stability of potent dipeptide sequences

Four predicted stable, neutral and instable dipeptides, respectively, were located within the primary structure of food proteins and were synthesized for validation of the stability clustering model. Stability of dipeptides was predicted with high accuracy as indicated by 9 correct predictions in the set of 12 dipeptides. The highest predictive error was observed for YG (Tyr-Gly) which was predicted instable but exhibited high stability under simulated intestinal conditions. Two predictive errors of stability were observed for the set of neutral dipeptides. Among the predicted dipeptides, SP (Ser-Pro) was the most intestinal stable dipeptide (Table 5).

Conclusion

The above experiments (i.e. in vitro and in silico) demonstrate that the following dipeptides exhibit both high intestinal stability as well as ACE inhibitory activity: DW, PW, GC and GN.

**Table 1:** Correlation coefficients for 17 stability and ACE inhibitory activity QSAR models for dipeptides based on different amino acid and peptide descriptors.

| Model[1] | Stability Correlation Coefficients ($R^2$) | Activity Correlation Coefficients ($R^2$) |
|---|---|---|
| 1 | 0.57 | -0.54 |
| 2 | 0.74 | -0.68 |
| 3 | 0.70 | -0.62 |
| 4 | 0.74 | -0.72 |
| 5 | 0.65 | -0.69 |
| 6 | 0.62 | -0.60 |
| 7 | 0.62 | -0.60 |
| 8 | 0.76 | -0.72 |
| 9 | 0.73 | -0.69 |
| 10 | 0.76 | -0.72 |
| 11 | 0.66 | -0.71 |
| 12 | 0.66 | -0.76 |
| 13 | 0.66 | -0.77 |
| 14 | 0.67 | -0.72 |
| 15 | 0.55 | -0.59 |
| 16 | 0.59 | -0.67 |
| 17 | 0.59 | 0.63 |

[1]Model 1 was created using a combination of physical chemical parameters and simple counts e.g. LogP, molecular weight, number of aromatic rings, number of rotatable bonds, polar surface area, number of H-bond acceptors and donors. Models 2-10 use individual and squared multivariate Z, X and H-scales and their products e.g. Z•X, Z•H and Z•H•X. For models 11-13 Extended Connectivity Fingerprints (ECFP) with different maximum diameters of 6, 10 and 12 were used, respectively. Model 13 is a combination of model 1 and 11. Model 15 uses E-state descriptors as defined by Kier and Hall. Models 16 and 17 were based on the best correlating descriptors; mainly hydrophobicity, shape, size and topological descriptors correlated with both activity and stability.

Model 16 was based on a Bayesian classification method while for model 17 a PLS analysis was done on the selected descriptors.

**Table 2:** Clustering of N- and C-terminal amino acid residues for contribution to small intestinal peptide stability[1].

| N-terminal AA residue | | | |
|---|---|---|---|
| *very stable* | *stable* | *neutral* | *instable* |
| Asp, Pro, Gly | Gln, His, Ile, Ser, Thr, Val, Asn, Glu | Tyr, Phe, Trp | Ala, Arg, Cys, Leu, Lys, Met |

| C-terminal AA residue | | |
|---|---|---|
| *very stable* | *neutral* | *instable* |
| Asp, Pro, Ser, Thr | Gly, His, Lys, Met, Asn, Glu, Gln, Val, Ile | Ala, Arg, Cys, Leu Phe, Trp, Tyr |

[1]The contribution of each individual amino acid residue to the observed overall peptide stability was calculated based on the average AUC of all peptides containing the amino acid residue of interest. The residues were subsequently grouped according to the mean calculated AUC.

**Table 3:** Grouping of dipeptide clusters into stable, neutral and instable dipeptides.

| Grouped cluster[1] | N-terminal cluster | C-terminal cluster |
|---|---|---|
| **Stable** | Very stable | Very stable |
| | Very stable | Neutral |
| | Very stable | Instable |
| | Stable | Neutral |
| | Stable | Very stable |
| | Neutral | Very stable |
| **Neutral** | Neutral | Neutral |
| | Instable | Very stable |
| **Instable** | Stable | Instable |
| | Neutral | Instable |
| | Instable | Instable |
| | Instable | Neutral |

[1]Based on experimentally measured small intestinal stability dipeptides were clustered according to their N- and C-terminal contribution to stability. N- and C-terminal amino acid clusters were plotted against each other and dipeptides were classified into stable, neutral and instable dipeptides.

**Table 4:** $IC_{50}$ values of selected dipeptides for ACE inhibition.[1]

| Peptide | $IC_{50}$ ($\mu mol \cdot L^{-1}$) means $\pm$ SE | | n | Peptide | $IC_{50}$ ($\mu mol \cdot L^{-1}$) means $\pm$ SE | | n |
|---|---|---|---|---|---|---|---|
| Ile-Trp | 19 $\pm$ 2 | | 2 | Trp-Ile | 100 $\pm$ 23 | | 2 |
| Glu-Trp | 21 $\pm$ 1 | | 2 | Trp-Leu | 105 $\pm$ 37.1 | | 2 |
| Ile-Tyr | 22 $\pm$ 4 | | 2 | Tyr-Trp | 107 $\pm$ 8.1 | | 2 |
| Val-Trp | 24 $\pm$ 7 | | 2 | Arg-Tyr | 113 $\pm$ 65.3 | | 2 |
| Lys-Pro | 40 $\pm$ 5 | | 2 | Trp-Tyr | 113 $\pm$ 0.8 | | 2 |
| Arg-Pro | 59 $\pm$ 6 | | 2 | Leu-Trp | 144 $\pm$ 36.6 | | 2 |
| Tyr-Tyr | 66 $\pm$ 31 | | 2 | Trp-His | 149 $\pm$ 0.5 | | 2 |
| Met-Trp | 76 $\pm$ 38 | | 2 | Met-Tyr | 151 $\pm$ 3.8 | | 2 |
| Val-Tyr | 81 $\pm$ 4 | | 2 | Tyr-Ile | 316 $\pm$ 19.1 | | 2 |

(continued)

| Peptide | IC$_{50}$ ($\mu$mol•L$^{-1}$) | | | Peptide | IC$_{50}$ ($\mu$mol•L$^{-1}$) | | | |
|---------|------|------|---|---------|------|------|------|---|
| | means $\pm$ SE | | n | | means $\pm$ SE | | | n |
| Ile-Phe | 92 | $\pm$ 10 | 2 | Asp-Lys | 510 | $\pm$ | 77.9 | 2 |

[1]ACE inhibition was measured in the absence and presence of increasing concentrations (2.5 - 500 $\mu$M) of dipeptide in the presence of the angiotensin-I like substrate Hip-His-Leu (1 mM). IC$_{50}$ values were calculated based on non-linear regression analysis. Results present mean $\pm$ SD from two independent IC$_{50}$ determinations.

**Table 5:** Prediction and experimental validation of ACE inhibitory dipeptides varying in intestinal stability.

| peptide | Stability | | | |
|---------|-----------|-----|-----------|-----|
| | predicted | | observed | |
| | overall | AUC | overall | AUC |
| Gly-Arg | stable | >4500 | stable | 4622$\pm$1283 |
| Trp-Pro | stable | >4500 | stable | 4606$\pm$129 |
| Tyr-Pro | stable | >4500 | neutral | 4187$\pm$399 |
| Ser-Pro | stable | >4500 | stable | 4887$\pm$639 |
| Trp-Gly | neutral | 1500 - 4500 | neutral | 4280$\pm$721 |
| Trp-His | neutral | 1500 - 4500 | neutral | 2934$\pm$800 |
| Trp-Asn | neutral | 1500 - 4500 | stable | 4812$\pm$901 |
| Tyr-Gly | neutral | 1500 - 4500 | neutral | 3384$\pm$475 |
| Tyr-Ala | instable | <1500 | stable | 4593$\pm$1930 |
| Arg-Trp | instable | <1500 | instable | 265$\pm$10 |
| Val-Tyr | instable | <1500 | instable | 373$\pm$27 |
| Asn-Tyr | instable | <1500 | instable | 240$\pm$12 |

Protein primary sequences were obtained from the ExPASy proteomics server of the Swiss Institute of Bioinformatics. Predicted stability refers to values obtained from cluster modelling while observed activity refers to the experimentally determined stability using custom synthesised peptides. AUC values represent mean $\pm$ SD, n=2.

**Supplemental Table 1:** Intestinal stability of dipeptides measured under simulated small intestinal conditions using in vitro digestion experiments (dissolution model). Stability is given as the integrated area of the peptide concentration under time curve (AUC) and ranges from 1 (0% stability at 1 min) to 6000 (100% stable over 60 min). Experiments were performed in duplicate and results are given as the mean.

| Peptide | AUC | Peptide | AUC | Peptide | AUC | Peptide | AUC | Peptide | AUC |
|---------|-----|---------|-----|---------|-----|---------|-----|---------|-----|
| FW | 269 | IM | 4937 | NV | 5766 | IY | 3163 | WD | 5331 |
| YE | 5630 | FP | 5559 | LT | 4801 | TM | 4648 | RR | 4489 |
| FH | 2339 | GD | 6000 | HL | 3060 | RP | 1933 | FR | 290 |
| MH | 1846 | GE | 5753 | EE | 5004 | YR | 463 | YK | 4365 |
| WV | 2983 | IG | 4051 | GF | 4562 | RK | 0 | IR | 5282 |
| ML | 361 | AM | 2578 | ST | 5706 | KI | 1317 | FL | 379 |
| VW | 2311 | NA | 4284 | SM | 5218 | ID | 5519 | MK | 1486 |
| NW | 1057 | GV | 6000 | YS | 5488 | LH | 2834 | MI | 842 |
| WR | 1209 | AN | 4400 | IA | 3993 | IK | 5520 | EI | 5905 |
| KW | 454 | VS | 5525 | LA | 3796 | PM | 5721 | ED | 4773 |
| HR | 0 | SV | 6000 | MS | 3668 | EN | 4902 | YQ | 5298 |

(continued)

| Peptide | AUC | Peptide | AUC | Peptide | AUC | Peptide | AUC | Peptide | AUC |
|---------|-----|---------|-----|---------|-----|---------|-----|---------|-----|
| TW | 777 | CG | 0 | DV | 5740 | WS | 5001 | HI | 5487 |
| WA | 3178 | IN | 5748 | VI | 5215 | PQ | 5892 | HM | 4884 |
| CW | 250 | GI | 6000 | IV | 5547 | LQ | 3210 | KH | 0 |
| TF | 291 | WW | 713 | IQ | 5301 | VF | 600 | NC | 0 |
| DE | 5648 | RM | 1831 | GK | 5971 | EP | 5905 | NF | 354 |
| IH | 5985 | YF | 250 | PC | 5298 | VN | 5270 | HD | 5291 |
| HT | 6000 | WQ | 4736 | GL | 4916 | LP | 5291 | AW | 3246 |
| GW | 5466 | YY | 250 | PA | 6000 | DI | 5950 | CT | 5502 |
| NQ | 4869 | IF | 2110 | TS | 4122 | CC | 2949 | HA | 5229 |
| FM | 2360 | HW | 2084 | LG | 2760 | EC | 0 | RA | 3647 |
| EY | 4095 | FN | 5300 | NT | 5422 | VL | 3390 | HF | 523 |
| RI | 1300 | DW | 5847 | PG | 5679 | MV | 3882 | AY | 931 |
| KR | 0 | RY | 1345 | TV | 6000 | TT | 5156 | MM | 1316 |
| HV | 5566 | RH | 0 | GQ | 5775 | IP | 5752 | FD | 5755 |
| LR | 1173 | YH | 3371 | KS | 5394 | TI | 5053 | TL | 2130 |
| VQ | 4625 | WE | 5000 | VA | 3963 | PV | 5964 | LD | 4232 |
| MC | 0 | YL | 844 | VV | 5766 | AD | 4298 | VT | 5700 |
| IE | 5234 | FK | 1601 | WI | 2471 | CN | 2028 | DL | 5322 |
| TN | 4990 | LK | 4409 | FF | 4453 | AP | 3783 | SL | 3600 |
| VH | 5173 | RV | 4584 | FE | 5019 | HS | 5881 | VP | 4877 |
| CK | 1917 | KE | 2854 | LW | 430 | GP | 5800 | IT | 5644 |
| FQ | 3736 | CF | 0 | FY | 352 | HG | 5451 | AF | 1700 |
| LM | 2574 | AR | 3508 | MW | 290 | VD | 5868 | LN | 3648 |
| MQ | 1610 | LY | 1744 | ER | 5539 | CA | 0 | MN | 3522 |
| NI | 5303 | MY | 250 | NM | 4984 | AE | 3548 | MT | 4819 |
| PY | 4776 | KF | 0 | RF | 4160 | WL | 1657 | IS | 5608 |
| LL | 891 | YD | 5696 | PK | 5499 | IW | 3967 | GA | 5966 |
| II | 5302 | KL | 613 | WK | 4603 | WT | 4403 | AT | 5274 |
| FS | 5437 | SW | 3413 | EM | 4110 | WY | 256 | DT | 5839 |
| SI | 5860 | ES | 4482 | MR | 261 | KM | 2763 | AS | 3975 |
| GM | 5047 | IL | 4330 | FI | 2044 | FV | 3293 | GG | 5810 |
| LV | 4424 | LE | 1984 | TY | 301 | LF | 473 | GN | 5648 |
| DR | 6000 | FT | 5139 | VR | 4074 | YM | 4244 | KA | 4312 |
|  |  |  |  |  |  |  |  | KP | 4051 |
|  |  |  |  |  |  |  |  | EV | 5981 |
|  |  |  |  |  |  |  |  | PD | 6000 |

**Supplemental Table 2:** Residual ACE activity in presence of 20 and 200 μM dipeptide was determined using an angiotensin-I like substrate (Hippuryl-His-Leu) in an at-line HPLC-UV assay. Means ± SE (n=2); n.d., not determined.

| Peptide | residual ACE activity (in % of control) | | | | | |
|---|---|---|---|---|---|---|
| | 20 μM dipeptide | | | 200 μM dipeptide | | |
| | mean ± SE | | n | mean ± SE | | n |
| AE | 96.4 | ± | 0.8 | 2 | 100.00 | ± | 0.0 | 2 |
| AF | 97.0 | ± | 3.0 | 2 | 86.50 | ± | 5.8 | 2 |
| AM | 95.7 | ± | 2.0 | 2 | 88.80 | ± | 4.3 | 2 |
| AN | 97.2 | ± | 2.8 | 2 | 99.05 | ± | 1.0 | 2 |
| AP | 88.6 | ± | 0.1 | 2 | 52.70 | ± | 2.7 | 2 |
| AR | 87.8 | ± | 1.8 | 2 | 67.95 | ± | 1.4 | 2 |
| AS | 95.4 | ± | 1.8 | 2 | 84.75 | ± | 2.3 | 2 |
| AW | 74.4 | ± | 6.2 | 2 | 30.50 | ± | 6.6 | 2 |
| AY | 89.7 | ± | 0.8 | 2 | 68.50 | ± | 6.2 | 2 |
| CA | 94.3 | ± | 5.1 | 2 | 36.75 | ± | 33.6 | 2 |
| CC | 59.6 | ± | 7.4 | 2 | 2.70 | ± | 0.3 | 2 |
| CF | 98.7 | ± | 1.3 | 2 | 52.35 | ± | 22.1 | 2 |
| CG | 98.9 | ± | 1.1 | 2 | 32.95 | ± | 27.5 | 2 |
| CK | 94.1 | ± | 5.0 | 2 | 88.80 | ± | 11.2 | 2 |
| CN | 100.0 | ± | 0.0 | 2 | 36.25 | ± | 18.4 | 2 |
| CT | 100.0 | ± | 0.0 | 2 | 34.70 | ± | 15.9 | 2 |
| CW | 99.4 | ± | 0.6 | 2 | 64.15 | ± | 29.7 | 2 |
| DE | 75.1 | ± | 11.0 | 2 | 60.45 | ± | 22.3 | 2 |
| DI | 72.3 | ± | 12.1 | 2 | 55.65 | ± | 21.9 | 2 |
| DL | 70.5 | ± | 8.8 | 2 | 51.50 | ± | 0.0 | 1 |
| DR | 49.8 | ± | 8.7 | 2 | 52.55 | ± | 19.7 | 2 |
| DT | 68.3 | ± | 3.6 | 2 | 68.50 | ± | 10.3 | 2 |
| DV | 67.0 | ± | 10.4 | 2 | 68.70 | ± | 9.3 | 2 |
| DW | 39.8 | ± | 8.5 | 2 | 58.00 | ± | 13.2 | 2 |
| EC | 77.8 | ± | 16.2 | 2 | 42.65 | ± | 3.8 | 2 |
| ED | 75.6 | ± | 10.6 | 2 | 79.30 | ± | 3.0 | 2 |
| EE | 78.7 | ± | 13.2 | 2 | 79.10 | ± | 5.4 | 2 |
| EI | 80.6 ± | | 0.0 | 1 | 65.40 | ± | 7.2 | 2 |
| EM | 68.9 | ± | 0.0 | 1 | 61.80 | ± | 0.0 | 1 |
| EN | n.d. | | | | n.d. | | | |
| EP | n.d. | | | | n.d. | | | |
| ER | n.d. | | | | n.d. | | | |
| ES | 64.9 | ± | 0.7 | 2 | 80.05 | ± | 4.0 | 2 |
| EV | 70.1 | ± | 4.5 | 2 | 78.70 | ± | 7.9 | 2 |
| EW | 34.1 | ± | 1.8 | 2 | 20.30 | ± | 1.3 | 2 |
| EY | 59.2 | ± | 0.0 | 1 | 54.20 | ± | 0.0 | 1 |
| FC | 100.0 | ± | 0.0 | 1 | 83.00 | ± | 0.0 | 1 |
| FD | 98.0 | ± | 0.0 | 1 | 100.00 | ± | 0.0 | 1 |
| FE | 99.4 | ± | 0.0 | 1 | 100.00 | ± | 0.0 | 1 |
| FF | 92.9 | ± | 0.0 | 1 | 81.90 | ± | 0.0 | 1 |
| FH | 100.0 | ± | 0.0 | 1 | 77.00 | ± | 0.0 | 1 |
| FI | 100.0 | ± | 0.0 | 1 | 91.20 | ± | 0.0 | 1 |

(continued)

| Peptide | residual ACE activity (in % of control) | | | | | | |
|---|---|---|---|---|---|---|---|
| | 20 μM dipeptide mean ± SE n | | | | 200 μM dipeptide mean ± SE n | | |
| FK | 83.4 | ± | 0.0 | 1 | 87.00 | ± | 0.0 | 1 |
| FL | 83.6 | ± | 0.0 | 1 | 84.60 | ± | 0.0 | 1 |
| FM | 100.0 | ± | 0.0 | 1 | 86.00 | ± | 0.0 | 1 |
| FN | 100.0 | ± | 0.0 | 1 | 100.00 | ± | 0.0 | 1 |
| FP | 100.0 | ± | 0.0 | 1 | 87.40 | ± | 0.0 | 1 |
| FQ | 100.0 | ± | 0.0 | 1 | 92.50 | ± | 0.0 | 1 |
| FR | 99.3 | ± | 0.0 | 1 | 70.30 | ± | 0.0 | 1 |
| FS | 100.0 | ± | 0.0 | 1 | 88.80 | ± | 0.0 | 1 |
| FT | 98.9 | ± | 0.0 | 1 | 100.00 | ± | 0.0 | 1 |
| FV | 100.0 | ± | 0.0 | 1 | 100.00 | ± | 0.0 | 1 |
| FW | 99.0 | ± | 0.0 | 1 | 55.50 | ± | 0.0 | 1 |
| FY | 93.5 | ± | 0.0 | 1 | 48.30 | ± | 0.0 | 1 |
| GA | 64.7 | ± | 4.5 | 2 | 51.50 | ± | 8.4 | 2 |
| GD | 59.4 | ± | 0.0 | 1 | 59.05 | ± | 1.8 | 2 |
| GE | 61.9 | ± | 0.0 | 1 | 56.80 | ± | 0.0 | 1 |
| GF | 58.4 | ± | 0.0 | 1 | 50.20 | ± | 0.0 | 1 |
| GG | 60.7 | ± | 0.6 | 2 | 53.90 | ± | 3.9 | 2 |
| GI | 57.4 | ± | 0.0 | 1 | 39.20 | ± | 0.0 | 1 |
| GK | 50.7 | ± | 0.1 | 2 | 41.25 | ± | 3.5 | 2 |
| GL | 57.0 | ± | 2.2 | 2 | 50.40 | ± | 2.4 | 2 |
| GM | 55.1 | ± | 5.1 | 2 | 49.25 | ± | 5.2 | 2 |
| GN | 55.0 | ± | 0.3 | 2 | 47.95 | ± | 6.0 | 2 |
| GP | 63.7 | ± | 0.3 | 2 | 28.65 | ± | 4.2 | 2 |
| GQ | 52.1 | ± | 0.3 | 2 | 44.60 | ± | 0.0 | 1 |
| GV | 54.6 | ± | 5.9 | 2 | 49.00 | ± | 2.4 | 2 |
| GW | 46.8 | ± | 5.4 | 2 | 33.25 | ± | 13.7 | 2 |
| HA | 99.6 | ± | 0.4 | 2 | 94.25 | ± | 0.9 | 2 |
| HC | 97.9 | ± | 2.2 | 2 | 82.40 | ± | 1.4 | 2 |
| HD | 93.9 | ± | 6.2 | 2 | 95.85 | ± | 1.7 | 2 |
| HF | 98.8 | ± | 1.2 | 2 | 83.80 | ± | 3.4 | 2 |
| HG | 100.0 | ± | 0.0 | 2 | 94.10 | ± | 1.5 | 2 |
| HI | 97.2 ± | ± | 2.8 | 2 | 91.75 | ± | 4.7 | 2 |
| HL | 99.6 | ± | 0.5 | 2 | 88.35 | ± | 2.5 | 2 |
| HM | 97.3 | ± | 2.7 | 2 | 96.65 | ± | 2.3 | 2 |
| HR | 97.6 | ± | 2.5 | 2 | 91.10 | ± | 4.2 | 2 |
| HS | 97.8 | ± | 2.2 | 2 | 96.80 | ± | 3.2 | 2 |
| HT | 98.8 | ± | 1.2 | 2 | 99.15 | ± | 0.8 | 2 |
| HV | 99.9 | ± | 0.1 | 2 | 97.50 | ± | 2.5 | 2 |
| HW | 81.3 | ± | 1.2 | 2 | 41.62 | ± | 4.0 | 2 |
| IA | 98.3 | ± | 1.7 | 2 | 89.70 | ± | 3.9 | 2 |
| ID | 100.0 | ± | 0.0 | 2 | 99.90 | ± | 0.0 | 2 |
| IE | 96.8 | ± | 0.4 | 2 | 99.95 | ± | 0.0 | 2 |
| IF | 73.0 | ± | 0.0 | 2 | 28.55 | ± | 2.4 | 2 |
| IG | 97.2 | ± | 0.3 | 2 | 93.75 | ± | 1.2 | 2 |
| IH | 93.5 | ± | 1.5 | 2 | 69.85 | ± | 2.3 | 2 |
| II | 99.2 | ± | 0.8 | 2 | 99.60 | ± | 0.4 | 2 |

(continued)

| Peptide | residual ACE activity (in % of control) | | | | | | |
|---|---|---|---|---|---|---|---|
| | 20 µM dipeptide<br>mean ± SE n | | | | 200 µM dipeptide<br>mean ± SE n | | |
| IK | 99.4 | ± | 0.6 | 2 | 95.50 | ± | 1.3 | 2 |
| IL | 100.0 | ± | 0.0 | 2 | 99.05 | ± | 0.7 | 2 |
| IM | 90.8 | ± | 2.3 | 2 | 59.95 | ± | 2.0 | 2 |

| Peptide | residual ACE activity<br>(in % of control) | | | | | | |
|---|---|---|---|---|---|---|---|
| | 20 µM dipeptide<br>mean ± SE | | | n | 200 µM dipeptide<br>mean ± SE | | n |
| IP | 81.9 | ± | 0.5 | 2 | 46.90 | ± | 1.5 | 2 |
| IQ | 98.0 | ± | 1.5 | 2 | 84.10 | ± | 0.3 | 2 |
| IR | 91.8 | ± | 0.3 | 2 | 67.00 | ± | 1.2 | 2 |
| IS | 98.9 | ± | 0.2 | 2 | 86.10 | ± | 0.6 | 2 |
| IT | 98.2 | ± | 1.8 | 2 | 98.35 | ± | 1.5 | 2 |
| IV | 99.8 | ± | 0.2 | 2 | 96.75 | ± | 0.7 | 2 |
| IW | 33.2 | ± | 0.6 | 2 | 20.25 | ± | 0.1 | 2 |
| IY | 34.6 | ± | 0.2 | 2 | 0.95 | ± | 1.0 | 2 |
| KA | 77.8 | ± | 2.8 | 2 | 49.15 | ± | 0.3 | 2 |
| KE | 88.4 | ± | 1.8 | 2 | 88.95 | ± | 2.0 | 2 |
| KF | 80.3 | ± | 0.7 | 2 | 47.75 | ± | 0.7 | 2 |
| KH | 90.5 | ± | 2.5 | 2 | 77.50 | ± | 0.8 | 2 |
| KI | 87.4 | ± | 1.7 | 2 | 78.60 | ± | 0.2 | 2 |
| KL | 88.1 | ± | 1.5 | 2 | 84.30 | ± | 0.0 | 1 |
| KM | 83.9 | ± | 1.7 | 2 | 71.20 | ± | 1.3 | 2 |
| KP | 64.3 | ± | 2.1 | 2 | 28.20 | ± | 0.4 | 2 |
| KR | 87.2 | ± | 1.8 | 2 | 75.55 | ± | 4.1 | 2 |
| KS | 84.6 | ± | 1.4 | 2 | 70.40 | ± | 1.2 | 2 |
| LA | 86.0 | ± | 0.0 | 1 | 83.50 | ± | 0.0 | 1 |
| LD | 87.9 | ± | 0.0 | 1 | 87.80 | ± | 0.0 | 1 |
| LE | 88.1 | ± | 0.0 | 1 | 85.90 | ± | 0.0 | 1 |
| LF | 100.0 | ± | 0.0 | 1 | 79.60 | ± | 0.0 | 1 |
| LG | 88.4 | ± | 0.0 | 1 | 87.30 | ± | 0.0 | 1 |
| LH | 100.0 | ± | 0.0 | 1 | 83.80 | ± | 0.0 | 1 |
| LK | 86.8 | ± | 0.0 | 1 | 84.60 | ± | 0.0 | 1 |
| LL | 81.9 | ± | 0.0 | 1 | 85.70 | ± | 0.0 | 1 |
| LM | 84.4 | ± | 0.0 | 1 | 84.00 | ± | 0.0 | 1 |
| LN | 85.3 | ± | 0.0 | 1 | 86.90 | ± | 0.0 | 1 |
| LP | 80.3 | ± | 0.0 | 1 | 65.50 | ± | 0.0 | 1 |
| LQ | 88.8 | ± | 0.0 | 1 | 87.30 | ± | 0.0 | 1 |
| LR | 85.2 | ± | 0.0 | 1 | 81.70 | ± | 0.0 | 1 |
| LT | 100.0 | ± | 0.0 | 1 | 88.30 | ± | 0.0 | 1 |
| LV | 84.5 | ± | 0.0 | 1 | 87.40 | ± | 0.0 | 1 |
| LW | 50.5 | ± | 0.0 | 1 | 23.00 | ± | 0.0 | 1 |
| LY | 68.4 | ± | 0.0 | 1 | 44.70 | ± | 0.0 | 1 |
| MC | 79.6 | ± | 7.3 | 2 | 69.70 | ± | 7.6 | 2 |
| MH | 80.6 | ± | 9.5 | 2 | 64.05 | ± | 1.1 | 2 |

(continued)

| Peptide | residual ACE activity (in % of control) | | | | | |
|---|---|---|---|---|---|---|
| | 20 μM dipeptide | | | 200 μM dipeptide | | |
| | mean | ± | SE | n | mean | ± | SE | n |
| MI | 82.1 | ± | 10.0 | 2 | 79.80 | ± | 9.6 | 2 |
| MK | 77.5 | ± | 5.8 | 2 | 72.15 | ± | 8.9 | 2 |
| ML | 81.6 | ± | 9.9 | 2 | 77.75 | ± | 8.8 | 2 |
| MM | 77.0 | ± | 8.2 | 2 | 76.05 | ± | 8.4 | 2 |
| MN | 81.7 | ± | 10.7 | 2 | 83.00 | ± | 9.1 | 2 |
| MQ | 81.0 | ± | 11.8 | 2 | 79.25 | ± | 10.1 | 2 |
| MR | 74.7 | ± | 11.1 | 2 | 54.30 | ± | 7.1 | 2 |
| MS | 74.9 | ± | 5.5 | 2 | 74.05 | ± | 8.6 | 2 |
| MT | 73.4 | ± | 4.2 | 2 | 82.25 | ± | 11.4 | 2 |
| MV | 69.9 | ± | 1.2 | 2 | 80.90 | ± | 8.3 | 2 |
| MW | 44.2 | ± | 2.1 | 2 | 20.75 | ± | 1.3 | 2 |
| MY | 57.8 | ± | 1.8 | 2 | 28.25 | ± | 12.2 | 2 |
| NA | 97.8 | ± | 1.2 | 2 | 96.55 | ± | 0.6 | 2 |
| NC | 95.5 | ± | 0.5 | 2 | 92.95 | ± | 2.4 | 2 |
| NF | 95.9 | ± | 3.6 | 2 | 100.00 | ± | 0.0 | 2 |
| NI | 96.1 | ± | 1.5 | 2 | 96.60 | ± | 3.4 | 2 |
| NM | 98.2 | ± | 1.8 | 2 | 99.25 | ± | 0.7 | 2 |
| NQ | 96.7 | ± | 3.3 | 2 | 100.00 | ± | 0.0 | 2 |
| NT | 96.9 | ± | 3.1 | 2 | 97.25 | ± | 2.8 | 2 |
| NV | 97.3 | ± | 2.8 | 2 | 100.00 | ± | 0.0 | 2 |
| NW | 86.4 | ± | 4.7 | 2 | 66.45 | ± | 3.0 | 2 |
| PA | 88.2 | ± | 11.9 | 2 | 88.10 | ± | 11.9 | 2 |
| PC | 87.3 | ± | 12.8 | 2 | 85.60 | ± | 14.4 | 2 |
| PD | 87.1 | ± | 13.0 | 2 | 89.35 | ± | 10.7 | 2 |
| PG | 86.4 | ± | 13.7 | 2 | 88.15 | ± | 11.9 | 2 |
| PI | 86.5 | ± | 13.6 | 2 | 83.05 | ± | 16.5 | 2 |
| PK | 87.2 | ± | 12.8 | 2 | 86.25 | ± | 13.8 | 2 |
| PM | 89.5 | ± | 10.5 | 2 | 88.30 | ± | 11.7 | 2 |
| PQ | 89.2 | ± | 10.9 | 2 | 89.30 | ± | 10.7 | 2 |
| PR | 88.9 | ± | 11.2 | 2 | 87.15 | ± | 12.9 | 2 |
| PV | 88.7 | ± | 11.3 | 2 | 89.40 | ± | 10.6 | 2 |
| PY | 88.1 | ± | 11.9 | 2 | 87.20 | ± | 12.8 | 2 |
| RA | 86.4 | ± | 3.0 | 2 | 46.65 | ± | 1.2 | 2 |
| RF | 88.0 | ± | 3.0 | 2 | 61.35 | ± | 4.1 | 2 |
| RH | 93.1 | ± | 0.5 | 2 | 72.45 | ± | 4.3 | 2 |
| RI | 97.3 | ± | 2.7 | 2 | 89.05 | ± | 7.5 | 2 |
| RK | 98.2 | ± | 1.8 | 2 | 99.10 | ± | 0.9 | 2 |
| RM | 95.8 | ± | 1.5 | 2 | 87.05 | ± | 5.1 | 2 |
| RP | 61.3 | ± | 2.3 | 2 | 21.90 | ± | 2.4 | 2 |
| RR | 96.6 | ± | 3.4 | 2 | 91.75 | ± | 5.3 | 2 |
| RV | 97.5 | ± | 2.5 | 2 | 96.45 | ± | 3.5 | 2 |
| RY | 77.5 | ± | 2.6 | 2 | 20.90 | ± | 17.9 | 2 |

(continued)

| Peptide | residual ACE activity (in % of control) | | | | | |
|---|---|---|---|---|---|---|
| | 20 µM dipeptide | | | 200 µM dipeptide | | |
| | mean | ± | SE | n | mean | ± | SE | n |
| SC | 76.1 | ± | 23.9 | 2 | 85.25 | ± | 14.8 | 2 |
| SI | 88.7 | ± | 11.4 | 2 | 87.80 | ± | 12.2 | 2 |
| SL | 83.0 | ± | 17.0 | 2 | 86.90 | ± | 12.8 | 2 |
| SM | 90.2 | ± | 9.8 | 2 | 72.60 | ± | 23.7 | 2 |
| ST | 90.4 | ± | 9.7 | 2 | 78.70 | ± | 21.3 | 2 |
| SV | 89.6 | ± | 10.5 | 2 | 90.00 | ± | 10.0 | 2 |
| SW | 76.5 | ± | 11.8 | 2 | 45.25 | ± | 10.3 | 2 |
| TF | 89.7 | ± | 8.4 | 2 | 78.15 | ± | 9.5 | 2 |
| TI | 87.1 | ± | 4.5 | 2 | 85.90 | ± | 7.3 | 2 |
| TL | 87.4 | ± | 5.2 | 2 | 78.45 | ± | 6.0 | 2 |
| TM | 88.4 | ± | 8.9 | 2 | 84.40 | ± | 9.4 | 2 |
| TN | 80.1 | ± | 15.6 | 2 | 88.35 | ± | 3.3 | 2 |
| TS | 90.3 | ± | 9.8 | 2 | 83.80 | ± | 5.8 | 2 |
| TT | 91.7 | ± | 8.3 | 2 | 89.30 | ± | 6.4 | 2 |
| TV | 87.3 | ± | 12.8 | 2 | 89.80 | ± | 6.3 | 2 |
| TW | 78.3 | ± | 12.3 | 2 | 46.50 | ± | 4.9 | 2 |
| TY | 79.7 | ± | 11.0 | 2 | 59.25 | ± | 4.2 | 2 |
| VA | 69.5 | ± | 30.6 | 2 | 58.35 | ± | 17.1 | 2 |
| VD | 78.6 | ± | 21.4 | 2 | 100.00 | ± | 0.0 | 2 |
| VF | 71.6 | ± | 28.4 | 2 | 55.45 | ± | 6.0 | 2 |

| Peptide | residual ACE activity (in % of control) | | | | | |
|---|---|---|---|---|---|---|
| | 20 µM dipeptide | | | 200 µM dipeptide | | |
| | mean | ± | SE | n | mean | ± | SE | n |
| VH | 77.1 | ± | 22.9 | 2 | 67.90 | ± | 23.8 | 2 |
| VI | 73.8 | ± | 26.2 | 2 | 72.90 | ± | 27.1 | 2 |
| VL | 65.5 | ± | 29.6 | 2 | 63.40 | ± | 26.6 | 2 |
| VN | 100.0 | ± | 0.0 | 2 | 79.25 | ± | 20.8 | 2 |
| VQ | 68.5 | ± | 23.3 | 2 | 77.40 | ± | 21.3 | 2 |
| VR | 74.9 | ± | 21.1 | 2 | 46.10 | ± | 20.8 | 2 |
| VS | 78.1 | ± | 22.0 | 2 | 74.95 | ± | 25.1 | 2 |
| VT | 73.9 | ± | 26.2 | 2 | 73.75 | ± | 26.3 | 2 |
| VV | 72.8 | ± | 27.3 | 2 | 78.25 | ± | 21.8 | 2 |
| VW | 39.3 | ± | 17.3 | 2 | 19.45 | ± | 8.1 | 2 |
| WA | 82.6 | ± | 2.0 | 2 | 54.70 | ± | 1.6 | 2 |
| WD | 88.3 | ± | 1.7 | 2 | 90.30 | ± | 1.0 | 2 |
| WE | 85.5 | ± | 2.2 | 2 | 90.90 | ± | 0.9 | 2 |
| WI | 67.2 | ± | 4.4 | 2 | 11.30 | ± | 6.6 | 2 |
| WK | 82.3 | ± | 3.2 | 2 | 69.05 | ± | 1.5 | 2 |
| WL | 52.9 | ± | 1.6 | 2 | 9.00 | ± | 0.1 | 2 |
| WQ | 86.3 | ± | 0.8 | 2 | 60.75 | ± | 0.5 | 2 |
| WR | 76.7 | ± | 6.1 | 2 | 38.95 | ± | 5.1 | 2 |

(continued)

| Peptide | residual ACE activity (in % of control) | | | | | |
|---|---|---|---|---|---|---|
| | 20 $\mu$M dipeptide | | | 200 $\mu$M dipeptide | | |
| | mean $\pm$ SE | | n | mean $\pm$ SE | | n |
| WS | 86.5 $\pm$ | 4.3 | 2 | 54.55 $\pm$ | 3.4 | 2 |
| WT | 89.0 $\pm$ | 0.1 | 2 | 75.80 $\pm$ | 3.9 | 2 |
| WV | 85.4 $\pm$ | 4.8 | 2 | 54.00 $\pm$ | 1.6 | 2 |
| WW | 75.7 $\pm$ | 2.0 | 2 | 31.55 $\pm$ | 9.7 | 2 |
| WY | 61.5 $\pm$ | 2.4 | 2 | 13.85 $\pm$ | 11.5 | 2 |
| YD | 86.2 $\pm$ | 1.4 | 2 | 88.65 $\pm$ | 3.3 | 2 |
| YE | 89.5 $\pm$ | 0.0 | 2 | 89.45 $\pm$ | 4.4 | 2 |
| YF | 78.1 $\pm$ | 1.5 | 2 | 40.10 $\pm$ | 5.4 | 2 |
| YH | 80.9 $\pm$ | 0.6 | 2 | 43.30 $\pm$ | 13.6 | 2 |
| YK | 85.7 $\pm$ | 2.3 | 2 | 77.10 $\pm$ | 6.0 | 2 |
| YL | 78.0 $\pm$ | 1.6 | 2 | 42.80 $\pm$ | 7.3 | 2 |
| YM | 82.6 $\pm$ | 2.7 | 2 | 61.55 $\pm$ | 1.0 | 2 |
| YQ | 83.3 $\pm$ | 0.4 | 2 | 75.35 $\pm$ | 4.5 | 2 |
| YR | 78.5 $\pm$ | 2.3 | 2 | 48.95 $\pm$ | 2.0 | 2 |
| YS | 83.2 $\pm$ | 2.9 | 2 | 81.05 $\pm$ | 3.1 | 2 |
| YT | 85.1 $\pm$ | 4.0 | 2 | 80.50 $\pm$ | 1.6 | 2 |
| YY | 66.4 $\pm$ | 0.5 | 2 | 20.30 $\pm$ | 0.9 | 2 |

**Claims**

1. Food product suitable for the prevention or treatment of hypertension comprising one or more peptides wherein the N-terminal amino acid is D, G or P and wherein said peptides are selected from the group consisting of the dipeptides DW, PW, GC and GN.

2. Food product according to claim 1 wherein said peptide is a dipeptide.

3. Food product according to claim 1 or 2 wherein the amount of said peptide per total amount of serving of the food product is from 1 to 200 mg, preferably 2 to 100, more preferably 3 to 40 and most preferably 5 to 20 mg.

4. Food product according to any one of claims 1 to 3 comprising at least 1 mg/kg of said peptides, preferably from 10 to 200, more preferably from 20 to 100 and most preferably from 30 to 80 mg/kg of said peptides.

5. Food product according to any one of claims 1 to 4 being selected from the group consisting of drinks, preferably fruit juice products or dairy drinks, dairy products, frozen confectionary products and spreads or margarines.

6. Food product according to any one of claims 1 to 5 for use in the prevention or treatment of hypertension.

## Figure 1

# Figure 2

# Figure 3

Europäisches
Patentamt

European
Patent Office

Office européen
des brevets

# EUROPEAN SEARCH REPORT

Application Number

EP 08 16 3908

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE WPI Week 200570<br>Thomson Scientific, London, GB; AN 2005-677410<br>XP002514894<br>& CN 1 623 600 A (DALIAN INST CHEM PHYSICS CAS) 8 June 2005 (2005-06-08)<br>* abstract * | 1-6 | INV.<br>A61K38/05<br>A61P9/12 |
| A | EP 0 821 968 A (CALPIS FOOD IND CO LTD [JP] CALPIS CO LTD [JP])<br>4 February 1998 (1998-02-04)<br>* abstract *<br>* page 2, line 40 - line 48 *<br>* page 3, line 33 - line 49 * | 1-6 | |
| A | WO 2006/084560 A (UNILEVER NV [NL]; UNILEVER PLC [GB]; LEVER HINDUSTAN LTD [IN]; BREE AN) 17 August 2006 (2006-08-17)<br>* abstract *<br>* page 3, line 25 - line 28 *<br>* page 10, line 21 - line 29 * | 1-6 | |

TECHNICAL FIELDS
SEARCHED (IPC)

A61K
A61P

The present search report has been drawn up for all claims

| Place of search | Date of completion of the search | Examiner |
|---|---|---|
| Munich | 24 February 2009 | Bobkova, Dagmar |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 08 16 3908

This annex lists the patent family members relating to the patent documents cited in the above-mentioned European search report.
The members are as contained in the European Patent Office EDP file on
The European Patent Office is in no way liable for these particulars which are merely given for the purpose of information.

24-02-2009

| Patent document cited in search report | | Publication date | Patent family member(s) | | Publication date |
|---|---|---|---|---|---|
| CN 1623600 | A | 08-06-2005 | NONE | | |
| EP 0821968 | A | 04-02-1998 | AT | 374617 T | 15-10-2007 |
| | | | AT | 238825 T | 15-05-2003 |
| | | | DE | 69721407 D1 | 05-06-2003 |
| | | | DE | 69721407 T2 | 06-05-2004 |
| | | | DE | 69738185 T2 | 10-07-2008 |
| | | | DK | 1302207 T3 | 05-11-2007 |
| | | | DK | 821968 T3 | 02-06-2003 |
| | | | ES | 2291417 T3 | 01-03-2008 |
| | | | ES | 2196263 T3 | 16-12-2003 |
| | | | JP | 3364579 B2 | 08-01-2003 |
| | | | JP | 10095736 A | 14-04-1998 |
| | | | JP | 2003048849 A | 21-02-2003 |
| | | | US | 5854029 A | 29-12-1998 |
| WO 2006084560 | A | 17-08-2006 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Gerstein et al.** *The Lancet,* 2000, vol. 355, 253-259
- **Hata, Y et al.** *American Journal of Clinical Nutrition,* 1996, vol. 64, 767-771
- **van Platerink CJ ; Janssen HG ; Horsten R ; Haverkamp J.** Quantification of ACE inhibiting peptides in human plasma using high performance liquid chromatography-mass spectrometry. *J Chromatogr B Analyt Technol Biomed Life Sci,* 2006, vol. 830, 151-157

- **van Platerink CJ ; Janssen HG ; Haverkamp J.** Development of an at-line method for the identification of angiotensin-I inhibiting peptides in protein hydrolysates. *J Chromatogr B Analyt Technol Biomed Life Sci,* 2007, vol. 846 (1-2), 147-154
- **Hellberg S ; Sjostrom M ; Skagerberg B ; Wold S.** Peptide Quantitative Structure-Activity-Relationships, A Multivariate Approach. *J Med Chem,* 1987, vol. 30, 1126-1135